# EUROPEAN PATENT SPECIFICATION

(11) **EP 3 417 295 B1**
(45) Date of publication and mention of the grant of the patent: **01.01.2020**
(21) Application number: 17710484.1
(22) Date of filing: 17.02.2017
(51) Int. Cl.: G01N 33/68

(54) **METHOD FOR THE DIAGNOSIS AND TYPING OF AMYLOIDOSIS**
VERFAHREN ZUR DIAGNOSE UND TYPISIERUNG VON AMYLOIDOSE
PROCÉDÉ DE DIAGNOSTIC ET DE TYPAGE DE L'AMYLOÏDOSE

(30) Priority: 18.02.2016 IT UB20160863
(43) Date of publication of application: 26.12.2018
(73) Proprietor: Universita' Degli Studi Di Padova, 35122 Padova (IT)
(72) Inventor: CASTELLANI, Chiara, 35138 Padova (IT); ANGELINI, Annalisa, 35128 Padova (IT); VALENTE, Marialuisa, 35030 Rubano (IT); THIENE, Gaetano, 35030 Rubano (IT); FEDRIGO, Marny, 35030 Galzignano (IT)
(74) Representative: Zaccaro, Elisabetta
(86) International application number: PCT/EP2017/053662
(87) International publication number: WO 2017/140872

(56) References cited:
- FALK RODNEY H: "Diagnosis and management of the cardiac amyloidoses", CIRCULATION, LIPPINCOT WILLIAMS AND WILKINS, BALTIMORE, US, vol. 112, no. 13, 27 September 2005 (2005-09-27), pages 2047-2060, XP002485173, ISSN: 1524-4539, DOI: 10.1161/CIRCULATIONAHA.104.489187
- GIRISH C. MELKANI ET AL: "Huntington's Disease Induced Cardiac Amyloidosis Is Reversed by Modulating Protein Folding and Oxidative Stress Pathways in the Drosophila Heart", PLOS GENETICS, vol. 9, no. 12, 19 December 2013 (2013-12-19), page e1004024, XP055308997, DOI: 10.1371/journal.pgen.1004024
- Hirano Koji ET AL: "The Signification Of Lactoferrin In Secondary Corneal Amyloidosis", , 1 April 2011 (2011-04-01), XP055308794, Retrieved from the Internet: URL:http://iovs.arvojournals.org/article.a spx?articleid=2350910 [retrieved on 2016-10-07]
- J. GUAN ET AL: "Lysosomal dysfunction and impaired autophagy underlie the pathogenesis of amyloidogenic light chain-mediated cardiotoxicity", EMBO MOLECULAR MEDICINE, vol. 6, no. 11, 1 November 2014 (2014-11-01), pages 1493-1507, XP055308912, Weinheim ISSN: 1757-4676, DOI: 10.15252/emmm.201404190

## Description

### FIELD OF THE INVENTION

The invention relates to a method for diagnosis and chemical typing of amyloidosis, and, in particular, for the diagnosis and chemical typing of cardiac amyloidosis due to transthyretin (ATTR). The invention also relates to a lysis buffer for protein extraction and to a diagnostic kit.

### STATE OF THE ART

Amyloidosis is a disease characterized by extracellular deposition of insoluble proteinaceous material with reduced molecular weight, also known as amyloid due to properties, similar to those of the starch, of reacting with iodine. These deposits show an eosinophilic dyeing affinity with standard tissue staining; they form bonds with Congo Red and emit an apple-green birefringence when seen through a polarized light microscope. These deposits are characterized by the evidence of a series of unbranched fibrils, having size of 75 to 100 Å, that appear under an electron microscope. Unlike what was initially thought, the substance is not amorphous but has a β sheet structure.

The fibrils have a very diverse chemical composition, since the amyloidogenic peptide, that is the most external part of them (95%), results from the deposition of a precursor protein that is different depending on the disease in question. Instead, the amyloid is internally made up of a core of amyloid P or AP component ("amyloid P component"), globular glycoprotein, i.e. a natural component of basal membranes that has a blood precursor called SAP ("serum amyloid P component"), and aggregates of chondroitin sulfate and heparan sulfate.

The isolation of the proteins making up amyloid and their chemical characterization are essential tools to understand the disease.

Although the proteins involved in these diseases are not related to one another considering either structure or function, insoluble amyloid aggregates have surprisingly similar characteristics. In fact, all of them show fibrillar type morphology, an unchanged amyloid fibrils structural characteristic that consists of a predominant secondary "β-sheet" structure, insolubility in common solvents and detergents, high resistance to protease, apple-green birefringence after staining with Congo Red, and Thioflavin T binding ability. Amyloid fibrils are semi-flexible, typically linear, 6-12 nm wide structures; they are formed by a variable number, usually from 3 to 6, of extended filaments (protofilaments) of about 1-2 nm in diameter, coiled to form a "rope" structure. The growth of each protofilament proceeds bidirectionally by apposition of new oligomeric units, while lateral association of more protofilaments originates wide amyloid fibrils. As previously mentioned, the ordered "core" of the amyloid fibrils is formed by a "cross-β" structure, in which each protofilament is constituted by a double row of "β-sheets"; this is a secondary structure according to which each monomer, whose "β-strands" run parallel to one another and perpendicular to the main axis of the fibril, is organized.

A number of studies have clarified that the basis of this aggregation is a more or less partial "misfolding" of the proteins involved in the pathogenesis, and also that a growing number of evidence supports the idea that the ability to form fibrils is an intrinsic property of most of polypeptide chains, although they do not have any sequence homology, i.e. many proteins, or perhaps all of them, would be potentially capable of forming amyloid fibrils in appropriate destabilizing conditions. The proposed molecular basis of the protein aggregation is that the fibrillogenesis take place when the stiff native structure of a protein is in some way destabilized, and is therefore subjected to a partial "misfolding", as it loses its three-dimensional structure closely "packed". Under these "non-native" conditions the protein structure becomes loose and its hydrophobic regions become exposed to the solvent; this promotes a tendency to "nucleation", i.e. to formation of oligomeric assemblies with increased content of "β-sheets". These "aggregation cores" provide a sort of "template", able to recruit other partially re-bent molecules, thus increasing the size of growing filaments and possibly giving rise to fibrillar aggregates.

To date, more than 30 types of proteins are known, which are involved in the deposition of amyloid fibrils in different organs. However, the most common forms are the amyloidosis by deposition of immunoglobulin light chain (AL), reactive amyloidosis (AA) in which the fibrils are derived from monoclonal immunoglobulin light chains and serum amyloid A (SAA) acute phase protein, respectively, and amyloidosis derived from accumulation of transthyretin (ATTR), a protein circulating in the plasma, which percentage is particularly high in certain geographic areas.

Although the proteins involved in the amyloidogenic process are different, depending on the type of amyloidosis, and acquire the amyloidogenic power in different ways, they share characteristic conformational and dyeing properties: i) an amorphous substance appearance under normal light microscope; ii) a characteristic "apple-green" birefringence under polarized light microscopy with Congo Red staining; iii) an ultrastructure composed of fibrils variable in the range of 7 to 10 nm in diameter, consisting of amyloid and other additive factors such as proteoglycans, visible under the electron microscope; iv) a "β-pleated sheet" conformation visible with infrared spectroscopy.

Currently, the diagnosis of amyloidosis is structured in two successive steps: (a) histological finding of material deposits with apple-green birefringence under polarized light fluorescence microscopy observation, after staining with Congo Red and Thioflavin-T, and (b) chemical typing of deposits to identify the type of protein that has undergone a "misfolding" performed by immunoelectronmicroscopy. Therefore, evidence that amyloid is present in an organ is only the beginning of the diagnostic process, the identification of the type of protein that is the cause of the deposit being very important, as the therapeutic intervention is different based to the type of amyloid.

Immunohistochemical characterization based on the use of antibodies directed against different amyloidogenic protein is a technique that plays an essential role in the second diagnostic typing step.

However, immunohistochemistry is a technique that presents some problems due mainly to three events: 1) a weak staining; 2) a non-specific staining; and 3) a lack of staining (Satoskar et al. Am J Surg Pathol 2011).

A weak staining or lack of staining could lead to the presence of false negatives. This occurs mainly when anti K light chains and anti lambda light chains antibodies are used. This is due to the presence of light truncated chains in amyloid deposits, which lead therefore to an unclear or non-recognition of the antigen by the antibody. Non-specific staining, instead, results in the occurrence of false positives, and is essentially the result of a non-specific binding of the antibody to amyloid deposits. Currently, antibody sensitivity is at 38-65%, according to studies in literature (Lachmann HJ et al. N Engl J Med 2002, Novak L et al. Nephrol Dial Transplant 2004).

In particular, the problem is amplified in the case of a false positive in the presence of a strong staining for the anti-TTR antibody in the myocardium. The cause is not yet certain, but the best hypothesis is that the contractile function of the myocardium can attract the TTR protein (also called pre-albumin) in the same muscle from the circulatory system, and that this can build up amyloid deposits making them positive to antibody staining (Kieninger B et al. Virchow Arch 2010). Furthermore, in cases of TTR derived amyloid, immunohistochemistry fails to define and distinguish between senile TTR and genetically modified TTR, i.e. familiar TTR. Amyloid deposits can be formed from more than one protein and determination of the type of protein becomes difficult by exclusive use of immunohistochemistry.

An alternative technique most used in the diagnostic field is the immunogold, or electron microscopy applied immunohistochemistry. This is a very precise technique, but involves problems that are not always easy to solve, such as: the availability of a transmission electron microscope (TEM), the need for personnel trained in handling and preparing the samples, but, above all, capable of treating radioisotope material. Moreover, necessary times are long enough and not always compatible with real-time diagnostics.

Falk (Circulation, 2005), Girish et al. (PLOS Genetics, 2013), Hirano et al. (Arvojournals, 2016) and Guan et al. (EMBO, 2014) describe methods for identifying cardiac amyloidosis by immunoblot methodology.

It remains in any case so far, the most used technique for chemical characterization of amyloidosis, although it is evident that it is an expensive diagnostic procedure in terms of employed staff and in economic terms.

Recently, some groups have tried to identify the proteins that make up the fibrils of amyloid also by using mass spectrometry.

Proteomic techniques have also come into use, as they are considered more specific and sensitive on the biopsy sample compared to immunohistochemistry, which, however, despite its limitations, is still the routine technique in a pathology laboratory as compared to immunoelectronmicroscopy and the previously mentioned mass spectrometry. In fact, these latter techniques require not only tools and skilled technicians for implementation, but also time intervals that are not compatible with real-time diagnostics.

Therefore, there is a need to find an alternative methodology that is sensitive and specific and that, in case of doubtful result, lends itself to a quality evaluation in the field of chemical characterization, which in this case appears to be the mass spectrometry.

A simple and routinely applicable approach appears to be the protein extraction, followed by SDS-PAGE for the separation of proteins and western blot (immuno-blot) for chemical identification of the relevant protein.

However, the technique may have some problems arising in the protein extraction method. The problem becomes even more significant in the case of TTR protein, where presence or lack of the monomer gives an indication about its involvement in the formation of amyloid fibrils. In particular, protein extraction often determines extract degradations and/or reductions which cause the presence of the TTR pathological monomer also in the case of healthy samples.

Furthermore, with the extraction methods known in literature, a filtration and/or a change of the solvent is necessary before proceeding to the electrophoretic procedure.

It is therefore object of the present invention to provide a method of diagnosis and typing of amyloidosis, in particular of transthyretin correlated amyloidosis, which can be applied routinely in a pathological anatomy laboratory.

### SUMMARY OF THE INVENTION

For the aforesaid purpose the inventors have developed a method of diagnosis and typing according to which protein extraction is carried out in non-denaturing conditions, suitable to preserve the structure of the proteins themselves.

An object of the invention is therefore a method for the diagnosis of amyloidosis, comprising the steps of:
a. contacting a sample of biological material with a lysis buffer having a pH of 8.8 comprising 65.5 mM Tris-HCL, 10% w/v glycerol, 2.3% w/v SDS, and a 2X protease inhibitor;
b. sonicating the sample of step a. at 4°C for 5 minutes and leaving it to rest for another 5 minutes;
c. repeating step b. at least two more times;
d. centrifuging the sample of step c.;
e. recovering the supernatant resulting from the centrifuging step d., thus obtaining a sample of extracted proteins;
f. separating and analyzing proteins present in the sample obtained from step e. by means of an electrophoretic technique and a subsequent immunofixation (immunoblot).

According to the method of the invention, a suitable buffer had to be developed for extraction of the protein and, therefore, another object of the invention is the production of a lysis buffer having pH 8.8 and comprising 65.5mM Tris-HCL, 10% w/v glycerol, 2.3% w/v SDS, 2X protease inhibitor.

Yet another object of the invention is a kit comprising at least a container with the lysis buffer having a pH of 8.8 and comprising 65.5mM Tris-HCL, 10% w/v glycerol, 2.3% w/v SDS, and a 2X protease inhibitor, and an instructions leaflet.

These and other objects as well as features and advantages of the present invention will be better understood from the following detailed description.

### BRIEF DESCRIPTION OF THE DRAWINGS

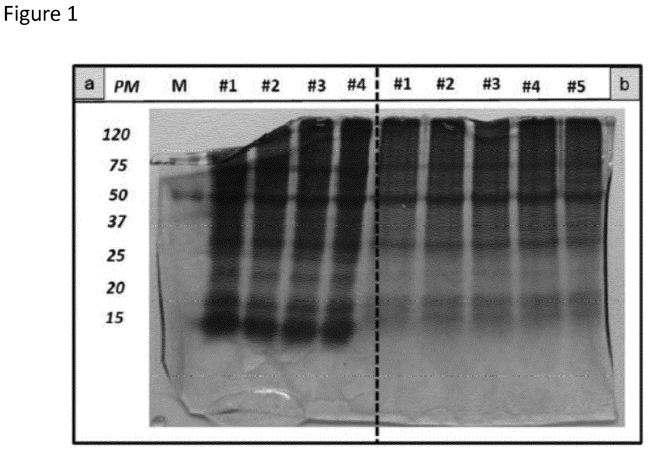

Figure 1. SDS-PAGE gel with Coomassie staining: the gel shows the electrophoretic run of protein extracts from frozen tissue on the right in a) and from paraffin treated tissue on the left in b). As it can be seen, a good protein extract yield can be obtained from tissue fixed in formalin and embedded in paraffin. 10 µg were loaded per well.

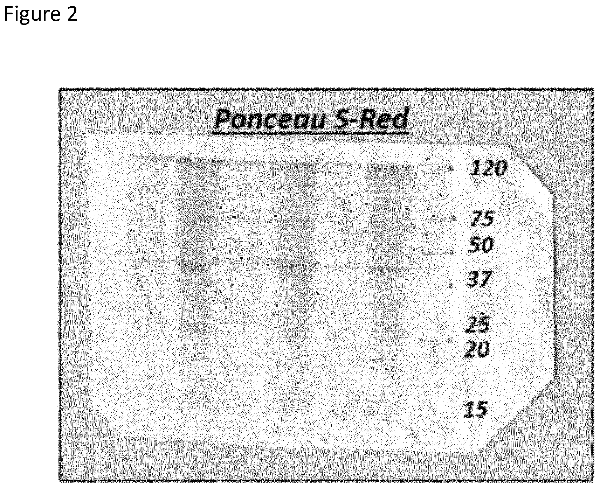

Figure 2. Ponceau S-Red staining: Western blot staining with Ponceau S-Red shows that the transfer of the protein extract containing the amyloid protein to a nitrocellulose membrane is very efficient. The protein bands in the electrophoresis gel are preserved even after the transfer to the membrane.

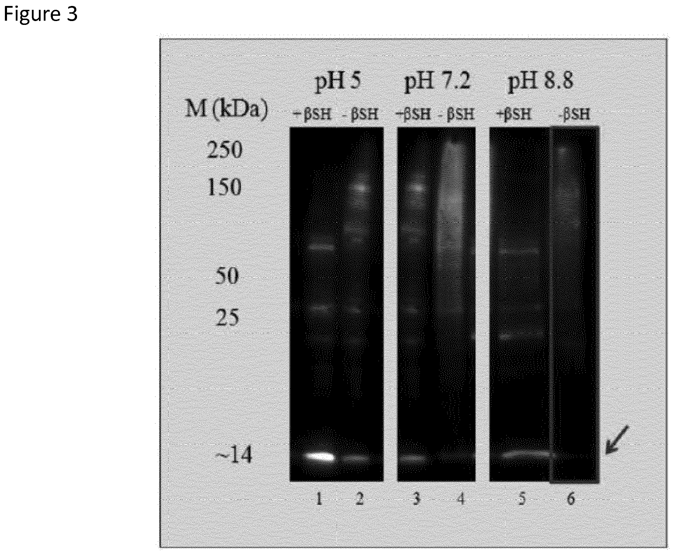

Figure 3. Western Blot for the TTR protein of patients with negative amyloidosis diagnosis: the TTR protein shows significant changes on the basis of sample processing conditions. With acid and neutral pH, false-positive bands are made evident at about 14 kDa (TTR monomers) in the negative controls. With pH 8.8, in the absence of βSH, the TTR monomer is not marked. Only an alkaline pH and the absence of additives properly preserve the sample from the formation of the TTR monomer [TTR, transthyretin or pre-albumin; βSH, 2β-mercaptoethanol; kDa, kilo Dalton].

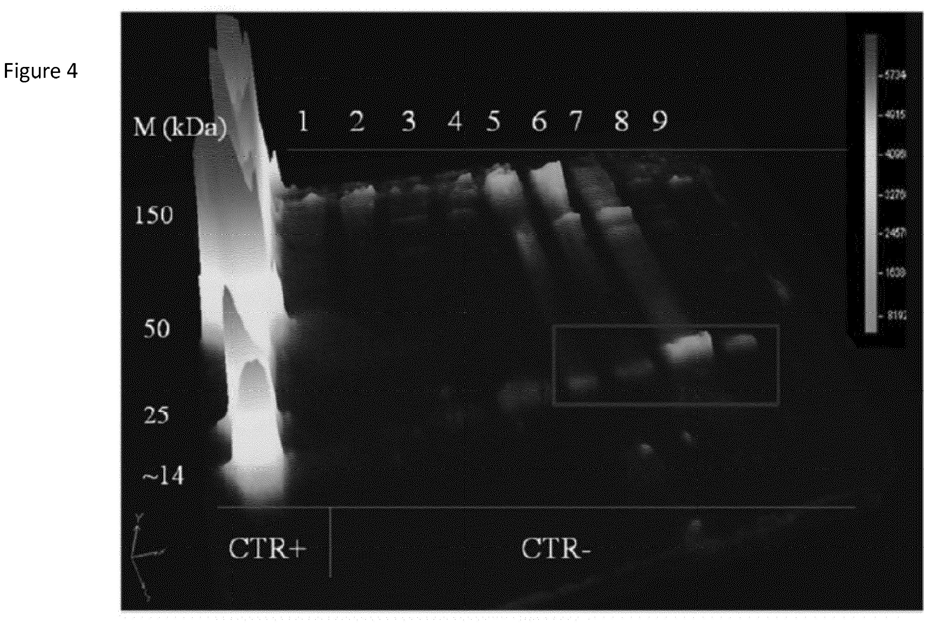

Figure 4. 3D Western Blot for TTR protein of patients with negative amyloidosis diagnosis for TTR extracts with different temperature conditions: The samples treated with β3H and temperature > 80°C show false-positive bands at about 14 kDa (TTR monomers, from line 5 to line 9). The samples, however, do not show the same peak intensity when compared to the positive control for TTR type amyloid shown on the left of the picture, the extraction of which was carried out under the same conditions. The samples treated at temperatures below 80°C, instead, do not show false-positive bands at about 14 kDa (TTR monomers, from line 1 to line 4).

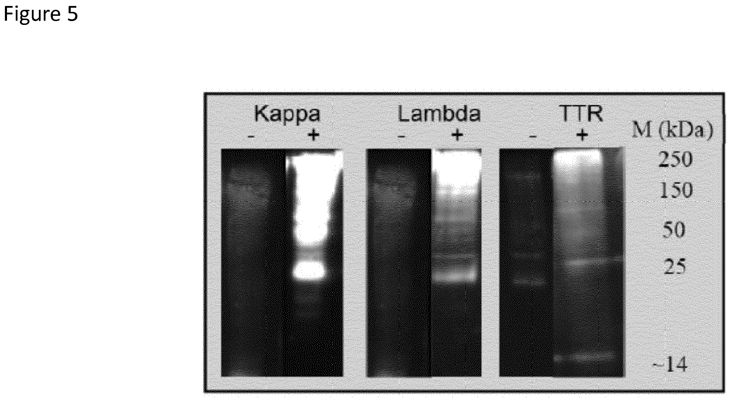

Figure 5. Western Blot of samples stored in RNAlater for k and lambda tight chains, and TTR protein: the Figure shows a representative selection of the chemical characterization findings with SDS-PAGE and Western Blot analysis of endomyocardial biopsies preserved in RNAlater.

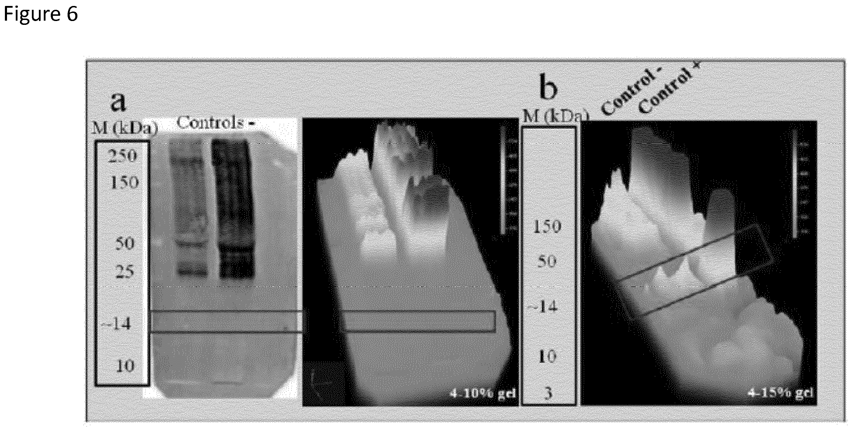

Figure 6. Western Blot of negative and positive samples for TTR cardiac amyloidosis. a) Classical and 3D view of Western Blot for the TTR protein in negative samples. A buffer with pH 8.8 without additives and with no temperature treatments preserves correctly from the formation *in vitro* of pathological TTR monomer. Both samples are negative and the diagnosis with immunoelectronmicroscopy confirms the data. b) Western Blot 3D view for the TTR protein in negative and positive samples for cardiac TTR amyloidosis. Both samples show a band at 14 kDa which corresponds to the pathological TTR monomer. The negative control shows a band intensity close to background noise.

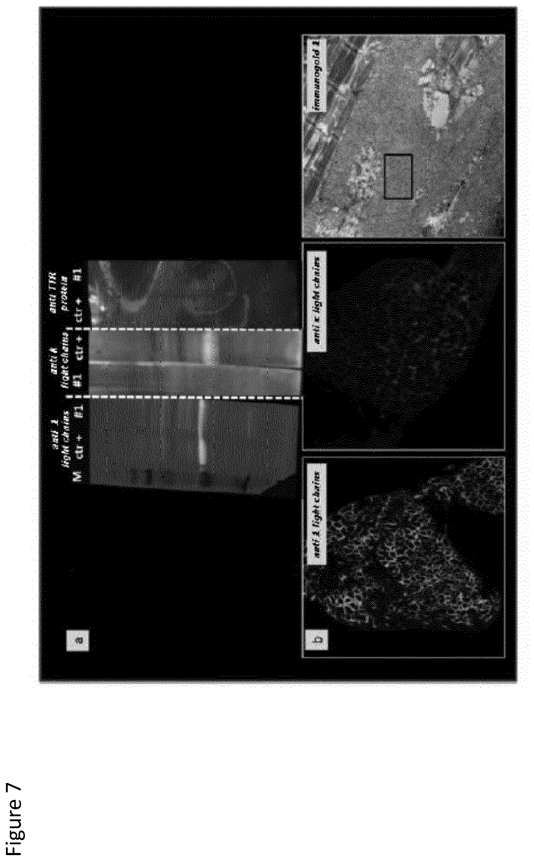

Figure 7. Western Blot of a sample with AL lambda (λ) type amyloid. a) positivity for anti λ light chains antibody in all samples; b) immunofluorescence for anti λ and κ light chain antibody. There is evidence of positivity for both antibodies. Immunoelectronmicroscopy shows positivity for λ light chains. Evidence of positivity is given by the presence of the anti λ light chains antibody conjugated with colloidal gold (in the box). 10,000X magnification.

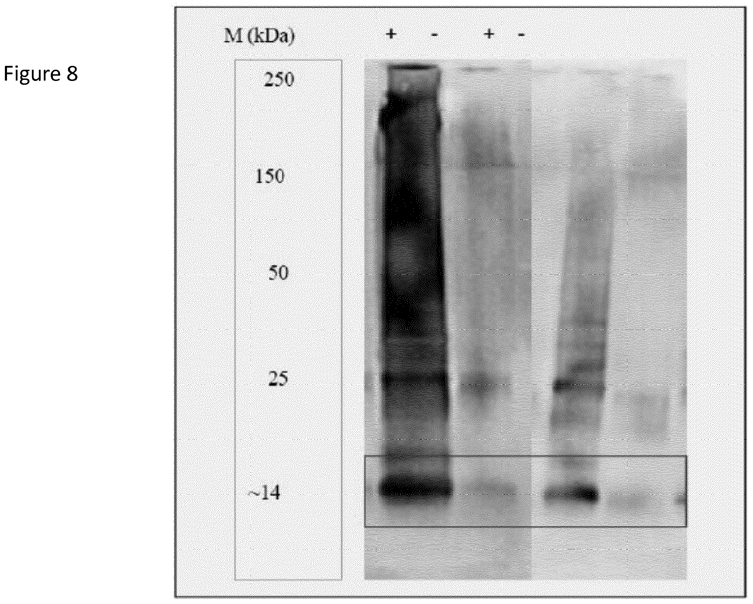

Figure 8. Western Blot in a case of TTR amyloid. Example of positive TTR amyloid. The Western Blot highlights positivity for the TTR antibody in the samples diagnosed with TTR amyloidosis through immunoelectronmicroscopy. Samples without the band at 14 kDa are of patients with negative diagnosis for TTR amyloidosis.

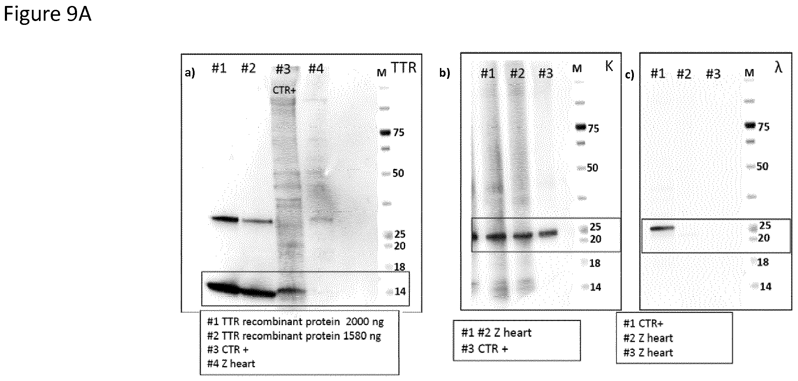

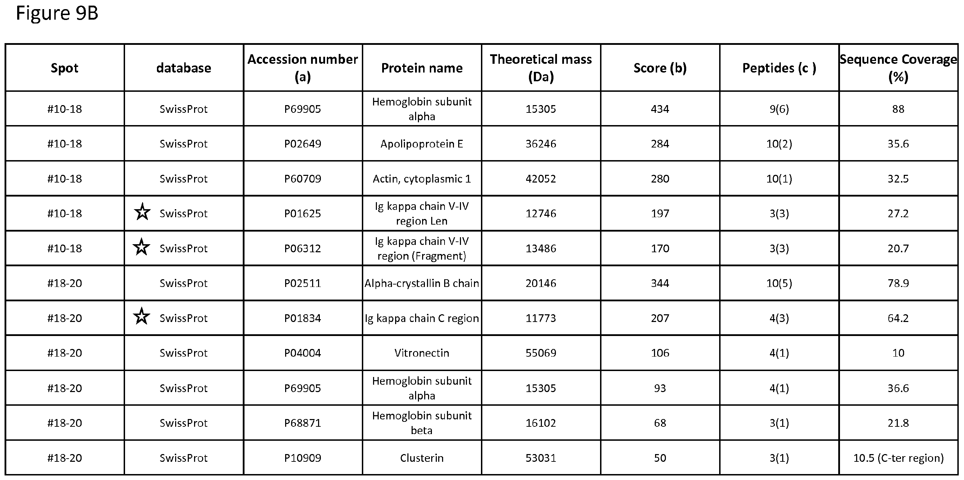

Figure 9. Mass Spectrometry of TTR amyloid. Figure 9A shows an example of a Western blot of the total protein extract from a sample of patient's heart tissue: a) Immunoblotting for the TTR monomer. Sample is TTR positive only when there is the presence of the TTR pathological monomer at lane 14-18 kDa (box).

Note how in the patient extract, lane 4, TTR monomer was not identified; b and c) Classical view of representative Western Blot for Lambda light chain (c) and Kappa light chain (b). Note how the sample is positive for K light chain (lane 1 and 2 in b, box) and negative for λ light-chain protein (lane 2 and 3 in c, box), indicating that the amyloid was an AL amyloidosis type k.

The legends to the lanes are reported in the figure for each gel, respectively.

Figure 9B reports the results of a representative scaffold readout of protein of interest for amyloid in the patient's heart sample. Mass spectrometry data show the presence of an Ig chain constant region and a light-chain variable region, with a probability of 95 %, indicating the presence of AL amyloidosis type K. The yellow stars indicate proteins of interest. The spot column indicates the molecular weight band of SDS-PAGE gel excised and processed by mass spectrometry

### DETAILED DESCRIPTION OF THE INVENTION

The present invention relates to a method for the diagnosis of amyloidosis.

In particular, a protein extraction protocol was devised that allows an exact chemical identification of the proteins involved in the formation of amyloid fibrils. Traditionally, isolation of the fibrils requires complicated extraction protocols, which often require high amounts of material.

The extraction and protein identification protocol according to the present invention has the advantages of allowing to start from small entities of material of various origins, which can be material previously stored with different methods. The developed protocol also allows extraction of amyloid protein components under conditions such that the protein is not denatured and is maintained as much as possible in its natural form. In cases of the transthyretin, it is the very presence of the monomer that gives indication of a pathological condition or not.

Currently, the immunohistochemical characterization requires the use of antibodies directed against different amyloidogenic proteins. However, immunohistochemistry for optical microscopy is not sufficiently sensitive and specific to allow an unambiguous typing of amyloidogenic deposits.

The present inventors have therefore developed and standardized a useful diagnostic technique for the chemical characterization of the fibrils deposited in the organs, which can be applied in clinical field.

The method for the diagnosis of amyloidosis according to the present invention comprises the steps of:
a. contacting a sample of biological material with a lysis buffer having a pH of 8.8, said buffer comprising 65.5 mM Tris-HCL, 10% w/v glycerol, 2.3% w/v SDS, 2X protease inhibitor, preferably a mixture of inhibitors proteases such as Protease Inhibitor Cocktail Tablets (Roche);
b. sonicating the sample of step a. at 4°C for 5 minutes and leaving it to rest for another 5 minutes;
c. repeating step b. at least 2-4 more times;
d. centrifuging the sample of step c.;
e. recovering the supernatant resulting from the centrifuging step d., thus obtaining a sample of extracted proteins;
f. separating and analyzing proteins present in the sample obtained from step e. by means of an electrophoretic technique and a subsequent immunofixation (immunoblot).

W/v or weight/volume refers to how many grams of solute are dissolved in 100 cm³ of solution.

The method according to the present invention has the advantage of being both sensitive and specific, and allows an optimization of the timing with which the final result is obtained. In fact, time for analysis is shortened from a month to 3 days from the electron microscopy without altering the specificity and sensitivity of the examination, and moreover the staff is not exposed to radioactive agents as usually happens with electron microscopy procedures.

Advantageously, the inventors have found experimental conditions that allow extraction of the proteins while maintaining their conformation and avoiding their degradation. They have found the best conditions of different factors among which pH, temperature, reducing agents and composition of the salts that make up the extraction buffer (buffer).

Surprisingly, the present method can be applied to any type of biological sample preservation manner, to fresh material but also to recycled material, preserved in RNAlater, which is a preservative usually used for the extraction procedures of genetic material (DNA and RNA). In this way, the patient does not have to undergo another type of procedure.

The starting material may also be in small amounts, as small as 5 mg.

In one embodiment, the invention relates to a method in which said biological sample has been previously stored by freezing, for example by means of "SNAP-freeze", in an OCT solution, in a solution that stabilizes the RNA, for example "RNAlater®", or by formalin fixation and paraffin embedding.

OCT (Optimal Cutting Temperature compound) consists in an aqueous solution of polyvinyl alcohol and polyethylene glycol, a substance that allows sample adhesion to the support and which facilitates cutting operation.

In a further preferred embodiment, the invention relates to a method in which said biological sample was previously preserved by means of formalin fixing and paraffin inclusion (FFPE).

Advantageously, the proteins can be extracted from formalin-fixed material and embedded in paraffin without having to start from large quantities, and therefore older biological samples can be used. Extraction kit already exist on the market, but they have substantially the problems related to the need to have high amounts of material. Moreover, often the extract is not compatible with mass spectrometry analysis. Small amount of material are sufficient for carrying out the present method.

In the embodiment including the biological sample being embedded in paraffin, a step c'. is added to the method, and a "dewaxing" step a'. is added before step a. The "dewaxing" step a' includes:
- adding 1 mL of xylene at room temperature, performing 4 washes of 15 minutes followed by 4 centrifugations between one wash and the other, 5 minutes at 12,000 rpm at room temperature, and discharge the xylene;
- adding 1 ml of absolute alcohol, performing 3 washes of 5 minutes followed by three centrifugations between one wash and the next one, 5 minutes at 12,000 rpm at RT, and dischargeing the alcohol, leaving the tube open under a hood to evaporate the residual alcohol.

In this case, the method accordingly comprises the steps of:
a'. treating a sample of biological material to remove the paraffin;
a. contacting a sample of biological material with a lysis buffer having a pH of 8.8, said buffer comprising 65.5 mM Tris-HCL, 10% w/v glycerol, 2.3% w/v SDS, 2X protease inhibitor,
b. sonicating the sample of step a. at 4°C for 5 minutes and leaving it to rest for another 5 minutes;
c. repeating step b. two-four times more;
c'. incubating the sample of step c. overnight in a thermostatic bath at 52°C;
d. centrifuging the sample of step c'.;
e. recovering the supernatant resulting from the centrifuging step d., thus obtaining a sample of extracted proteins; and
f. separating and analyzing the proteins present in the sample obtained from step e. by means of an electrophoretic technique and a subsequent immunofixation.

In one embodiment, according to the method of the present invention, the step of d. centrifuging the sample is preferably carried out at 4°C and 12000 rpm for 15 minutes.

In a further embodiment, the invention relates to a method wherein, before step a. of contacting the biological sample with the lysis buffer, said biological sample is washed with a saline to which a protease inhibitor is added, preferably sterile PBS with 1X protease inhibitors.

In a further preferred embodiment, the invention relates to a method for the diagnosis of amyloidosis, in particular of amyloidosis, preferably transthyretin related cardiac amyloidosis.

Advantageously, the inventors were able to find the conditions for identification of the TTR protein, in which the presence or absence of the monomer gives indications on its involvement in the formation of amyloid fibrils.

Previously, protein extraction often determined extract degradations and/or reductions which caused the presence of the TTR pathological monomer also in the case of healthy samples.

In a further embodiment, the invention relates to a method wherein the electrophoretic technique of said step f. is SDS-PAGE and the immunofixation technique is Western Blot.

The bands that are obtained from electrophoretic gels have the advantage that they can also be analyzed by mass spectrometry, so providing a "gold standard" to the procedure.

With the protein extract so obtained also a two-dimensional (2D) gel electrophoresis can be carried out, thus solving the complexity of the deposited proteins. Moreover, this allows detection and identification of changes in the expression of constitutive proteins of the tissue under examination related to the development of the disease.

The method according to the invention is simple and rapid in execution. As a matter of fact, the washing process of the frozen or preserved samples has an average duration of 2 hours. The subsequent step, that is the very extraction of the proteins from the tissue, has a duration variable from 2 hours to the duration of the night, depending on the extraction procedure choice. The quantification of the sample with the use of a standard kit, such as the Qubit® protein assay kit, takes about thirty minutes. The longest and more laborious steps are those relating to SDS-PAGE electrophoresis followed by Western Blot analysis, as these last up to 24 hours. Overall, three days are required to carry out all steps, including the acquisition and image analysis.

Advantageously, the present method can be applied to any biological sample or biopsy.

In a preferred embodiment, the biological sample used by the method is an endomyocardium biopsy of periumbilical fat.

The amyloid proteins deposit and accumulate in various tissues of the body. By accumulating in the kidneys, heart, liver, gastrointestinal tract or in the nerves, the amyloid causes a malfunction of these organs. Accordingly, symptoms of amyloidosis are associated with abnormal function of the organs involved. In general, amyloidosis is only one category of a growing list of disorders associated with protein folding. Different symptoms and life-threatening disorders manifest themselves depending on where the amyloid accumulates, such as kidneys, heart and nerves.

In a preferred embodiment, after the amyloidosis diagnosis, a typing of proteins is carried out.

Chemical level identification of the type of protein that causes the deposit is extremely important, because each type of amyloidosis progresses in different way and also the treatments are different. An accurate diagnosis and typing of amyloidosis are critical to determining the prognosis and drug treatment.

For the method of the invention, an appropriate buffer had to be developed for the extraction of proteins, and, therefore, another object of the invention is a lysis buffer having a pH of 8.8 and comprising 65.5mM Tris-HCL, 10% w/v glycerol, 2.3% w/v SDS, and 2X protease inhibitor, preferably a mixture of inhibitors proteases such as Protease Inhibitor Cocktail Tablets (Roche).

Yet another object of the invention is a kit comprising at least a container with the lysis buffer having a pH of 8.8 and comprising 65.5mM Tris-HCL, 10% w/v glycerol, 2.3% w/v SDS, and a 2X protease inhibitor, and an instructions leaflet.

The method of diagnosis and typing of amyloidosis has been validated with a study over heart amyloidosis due to transthyretin, hereinafter described in detail for purposes of illustration of the invention. In particular, the ability of SDS-PAGE electrophoresis and Western Blot to chemically characterize amyloid also in small amounts of tissue, such as endomyocardial biopsies, and starting from protein extracts of fresh biopsy samples was assessed. The samples were preserved in RNAlater or fixed with formalin and embedded in paraffin wax.

### EXPERIMENTAL SECTION

### Materials and methods

**Patients.** In the study all patients referred to the Unit of Cardiovascular Pathology U.O.C. and Patologic Anatomy and Histology CLOPD with with cardiac or systemic amyloidosis diagnosis were considered in the period 2011-2014. A total of 28 cases were included in the study. Patients were selected for type of amyloid according to the diagnosis carried out with "immunogold" and electron microscopy. A population homogeneous with the sample under exam as to age, sex, type of material was selected as control. The control population had not any diagnosis of amyloidosis, even in the past.

**Type of sample.** We used samples stored in OCT ("optimal cutting temperature compound"), in RNAlater, samples from SNAP-freeze or formalin-fixed and paraffin-embedded (FFPE).

**Organs tested by extraction:** endomyocardial biopsies, and periumbilical fat, **Protein extraction and treatment of the sample**

### Sample preparation procedure

### (a) Starting material stored in OCT, SNAP-freeze, RNA-later:

- from SNAP-freeze: cut a fragment of 2mmx2mm keeping it at -20°C;
- from OCT: cut sections 6 of 8 microns by a cryostat.
- from RNAlater (never used before for protein extraction): transfer the sample to a sterile tube, eliminating the preserving liquid.

Place the fragment, or the sections, in 1.5 ml sterile tubes and perform the following steps:
1. under a hood, wash 3 times for 5 minutes with sterile PBS and add 2X protease inhibitors (Complete Protease Inhibitor Cocktail Tablets, Roche). After each wash centrifuge the samples at +4°C for 15 minutes at 12,000 rpm and discharge the supernatant;
2. after the last wash, chop the sample with a disposable sterile blade, using a different blade for each sample;
3. add 200 µL of lysis buffer (based on the amount of sample to lyse) into the test tube containing the sample. The lysis buffer for protein extraction has a pH of 8.8 and comprising 65.5mM Tris-HCL, 10% w/v glycerol, 2.3% w/v SDS, and 2X protease inhibitor;
4. sonicate the sample 3 times at +4°C for 5 minutes. After each cycle make the samples rest at +4°C for 5 minutes;
5. centrifuge the sample for 15 minutes at +4°C at 12,000 rpm;
6. collect the supernatant containing the proteins in sterile 1.5 ml tubes;
7. store the sample at -80°C until use.

### (b) Starting material: small blocks of formalin fixed and paraffin-embedded material (FFPE blocks)

The steps performed on the fixed and included samples are:
1. cut 12 4mM sections from the microtome and place them in sterile 1.5 mL tubes. Use a different blade for each sample. Clean the blade and tweezers, by which you collect the slices, with alcohol between a sample and the other;
2. under the hood, proceed to dewaxing: add 1 mL xylene at room temperature and perform 4 washings of 15 minutes. Centrifuge for 5 minutes at 12,000 rpm at room temperature and discharge the xylene between one wash and the other;
3. add 1 mL absolute alcohol and perform 3 washes of 5 minutes. Centrifuge for 5 minutes at 12,000 rpm at RT and discharge the alcohol between one wash and the other;
4. after the last centrifugation, discharge the alcohol and leave the test tube open under a hood, in order for the excess alcohol to evaporate.
5. add 200 µL of lysis buffer with pH 8.8, which includes 65.5mM Tris-HCL, 10% w/v glycerol, 2.3% w/v SDS, 2X protease inhibitor into the test tube;
6. sonicate 5 times at +4°C for 5 minutes. After each cycle make the samples rest at +4°C for 5 minutes;
7. seal the tubes with parafilm (Parafilm M® All-Purpose Laboratory Film), place them on a support and incubate them in the static thermostat bath (critical tool) at +50-52°C overnight (o/n, critical step);
8. the next day centrifuge for 15 minutes at +4°C at 12,000 rpm;
9. collect the supernatant containing the proteins in sterile 1.5 ml tubes;
10. store the sample at -80°C until use.

From step 6 on, in to preserve the proteins as much as possible in their native form, the material must not undergo shaking either on shakers or on any other equipment. Therefore, the thermostatic bath should not include agitation mechanisms. Overnight incubation at 50-52°C allows the lysis buffer to solubilize the protein without excessively raising the temperature and then going to interfere with the protein structure. Over night time allows to obtain good protein concentrations in the buffer.

### Discussion

In order to be studied, the proteins must first be extracted from their biological matrix. The adopted strategy depends on a number of factors, such as the physical-chemical characteristics of the relevant protein, the desired quality of the extract and the amount of protein required. The procedures used to obtain a crude protein extract are extremely simple and require breaking the cells in a specific buffer (a lysis buffer having a pH of 8.8 and comprising 65.5mM Tris-HCL, 10% w/v glycerol, 2.3% w/v SDS, and 2X protease inhibitor) and the sample centrifugation to remove insoluble residues. In the specific case of this study, breaking of the cells immersed in the extraction buffer is obtained by repeated cycles of sonication. The sonicator is a tool that includes a probe capable of producing ultrasound. The high frequency ultrasound can be used effectively to break the cells, since the ultrasounds generate high pressure waves. The disadvantage of this technique is the generation of a significant overheating, that is why sonication is carried out in a frozen medium and the samples are kept at +4°C between a sonication cycle and another. At this point, each sample is subjected to various types of treatments including: changes in pH of the extraction buffer, conditions of work temperature (T) and addition or not of additives to the extraction buffer. Specifically we will have: 1) pH of the buffer: pH 5.0, pH 7.2 and pH 8.8; 2) additives added to the extraction buffer: 2β-Mercaptoethanol (β3H, Sigma-Aldrich, Milan, Italy); 3) work temperature: different work temperatures were tested in a range from 42°C to 80°C for 2 hours up to over night (o/n).

At the end of these treatments, each sample is centrifuged for the separation of the pellet from the supernatant which contains the proteins. The supernatant is divided into two parts. A part is used for protein quantification and quality control of the sample and another part is stored in a refrigerator at -20°C until ready to perform the SDS-PAGE and Western Blot tests.

**Protein quantification.** The extract proteins were quantified by fluorimetric detection. The equipment used is the Qubit® 2.0 Fluorometer (Invitrogen, Life Technologies, Monza, Italy) together with a specific kit for determining the amount of protein (Qubit® protein assay kit) and following the procedure described in the kit manual. The Qubit® protein assay kit was designed to quantify the amount of samples with a range of from 1:25 ug/mL to 25 ug/mL.

This type of kit can be very accurate even in the presence of contaminants possibly present in the samples, such as SDS and 2β-Mercaptoethanol.

### Protein separation.

### SDS-PAGE electrophoresis protocol

1. After selecting the relevant samples, the same are brought from -80°C to +4°C.
2. Prepare the portion for loading each sample, containing 12 µg of protein and 2 µL of bromophenol blue, which allows to monitor the electrophoresis run.
3. Use a gel (Criterion™ TGX Stain-Free™ Precast Gel, Bio Rad) for electrophoresis run, making sure to wash the wells prior to loading.
4. Pour the run solution in the cell for the electrophoretic run, so that the wells are covered. RUNNING BUFFER: 125mM Tris-HCL, 96mM Glycine, 0.5% w/v SDS.
5. Load a sample per well using a Hamilton® syringe. Wash the syringe with distilled water after the load of each run well.

Be careful to include a marker (10-250 kD) in each electrophoresis run.
6. Begin the electrophoresis run, following the instructions of the distributing company of the gel.
7. At the end of the run, recover the gel and balance it for 15 minutes at room temperature in the buffer for Western Blot.

### Discussion

The protein extract was subjected to electrophoresis on polyacrylamide gel in the presence of sodium dodecyl sulphate (SDS-PAGE) using gel with a concentration gradient of acrylamide variable between 10% and 20%. Only Criterion TGX gels, which are pre-cast gel supplied by the company Bio-Rad, are generally used. About 10-12 µg of sample were loaded per well. Alternatively, hand-made 4-10% and 4-20% gel can be used, by polymerizating them with the addition of Temed and 10% APS (Sigma Aldrich, Milan, Italy) at the time of use.

A voltage was then applied at the ends of the gel, so as to allow the proteins to migrate through the gel so prepared. For pre-cast gels the run conditions are provided by the company itself. For hand-made gels the conditions that must be met during the electrophoretic run are the following: 1) running buffer: 125 mM TRIS-HCI, 96 mM Glycine, 0.5% w/v SDS; 2) run conditions: 15 mA (before passing the "stacking gel") and 30 mA (up to dye leaking). The determination of the approximate molecular weight of the proteins was obtained by comparison with protein standards. After completion of the electrophoretic run, the gel was left 40 minutes Coomassie Blue to verify the presence and the quality of the migration of the bands. We have then proceeded by treating the gel with a wash solution, consisting of methanol, acetic acid and distilled water, in order to decolorize the gel, for the time necessary to obtain a good resolution of the protein bands. The gel was then photographed and stored in 5% acetic acid.

### Immuno-blot.

### Western blot procedure

1. After balancing the gel, proceed with the Western Blot, using the Trans-Blot® Turbo™ Transfer System and the Trans-Blot® Turbo™ Transfer Packs. Follow the Bio Rad instructions, selecting the 7 minutes protocol (for proteins from 5 to 150 kD).
2. After the procedure, proceed to blocking with 5% milk in TBS for 2 hours.
3. At the end of 2 hours, prepare the antibodies according to the manufacturer's instructions and incubate at +4°C over night.
4. The following day, proceed with the development of the membrane using Pierce ECL Western Blotting Substrate.
   BUFFER FOR WESTERN BLOT: 3 g Tris, 14.4 g Glycine, 200 ml methanol. Add distilled water up to 1L

### Discussion

After the electrophoretic run, proteins which are separated according to their molecular weight were transferred from the gel to a nitrocellulose membrane by "electroblotting". The Trans-Blot® Turbo™ Transfer Packs have everything needed for the transfer included in the package. The transfer was of "semi-dry" type (nitrocellulose from Amersham company). The blot was performed by using the Trans-Blot transfer System machine (BioRad, Milan, Italy). Alternatively, the same results can be obtained by using the classic "wet" method. In this case the transfer is achieved by applying a constant electric voltage of 100 Volts, with a current of 360 mA, for 1 hour 30 minutes in a cold room, in a basic pH environment. The migration of the proteins towards the anode, as most of the proteins are negatively charged with a basic pH, has been optimized by increasing the negative charge with low concentration SDS (less than 1%). The uniformity and the actual transfer of proteins from the gel to the membrane were checked by staining the same with Ponceau S (solution at 0.1% in 1% acetic acid). The membrane was then decolorized with distilled water. Optionally, the residual polyacrylamide gel can be colored with Coomassie blue to verify the efficiency of the transfer. Once the membrane is decolorized, the "blot" was immersed in a solution of 5% milk in 1X TBS to buffer the antigenic sites. The membrane was incubated with specific antibodies directed to the relevant protein. The exceding antibody was eliminated by means of washings, leaving the antibody that is associated with the protein. The antigen binding was revealed by developing the membrane.

The antibodies used in the study are: anti human k light chain, anti human lambda light chain and anti human transthyretin or TTR (Dako, Milan, Italy).

All blots were developed by chemiluminescence (Chemiluminate Kit, Delchimica, Naples, Italy, or ECL West Dura, Pierce).

**Image acquisition and analysis.** Detection of the bands was obtained by introducing the membranes in a suitably equipped photo acquisition device with relevant filters (2.7 Alliance, UVitec, Eppendorf Italy, Milan, Italy). Image analysis was performed with the Alliance 1 D software, which allows for taking even pictures in 3D. The acquisition and analysis software Alliance 1D allows, besides image acquisition, also calculation of molecular weights, electrophoretic distances and quantification of the peaks. The "Dynamic Image Technology" function shows directly the grayscale values of the image being captured, with saturation signs visible on the PC monitor for optimal and immediate image acquisition (for quantitative analysis). The "Image Display Technology" function improves the scanned image from the graphic presentation point of view without changinh/altering the original quantity data. The 3D reconstruction provides direct information regarding the dynamic range of the signals (16-bit scale, 65536 gray levels), the level of "background" of the image and the amount of protein of the acquired samples: quantification is based on the gray level values of all the pixels that make up the image. In the case of 3D real time visualization, small adjustments of exposure time lead to automatic updating of the 3D visualization itself. Also the saturation effect can be controlled in real time before the image is captured and saved. A color palette can be defined and applied for the 3D image, which allows you to apply the corresponding RGB (Red, Green, Blue) values specific for monochrome images. The pseudo colors can be viewed at different types or levels of signal intensity in an image. This color representation replaces the original gray scale levels for a more immediate visual effect.

### Results:

From the samples examined for the different types of storage, a quantity of protein could be extracted that could be used for SDS-PAGE electrophoresis as the optimal protein concentration cut-off is 0.9 µg/µL, which allows to load each well of the gel electrophoresis with about 12 µg of proteins. Furthermore, it was found that all the biopsy samples (control group + patients) preserved in RNAlater allow a more effective extraction yield. From the material fixed with formalin and paraffin-embedded (FFPE) we have been always able to extract a significant amount of protein within a range of 3.78 µg/µL to 1.21 µg/µL, which therefore is sufficient for the electrophoretic run. The SDS-PAGE electrophoresis of the protein extract obtained from the material fixed and embedded in paraffin was qualitatively comparable with that obtained from frozen material or material from RNAlater. Even the reference molecular weights were congruent with those obtained from the frozen material, as shown by the gel Coomassie staining in Figure 1.

The protein extract containing the amyloid protein presented an excellent efficiency in the transfer to nitrocellulose membrane. This is made evident by the staining with "Ponceau Red" after immunoblotting, which showed the same bands that were present in the gel electrophoresis (Figure 2).

The extraction buffer pH and the absence of reducing agents, such as 2β-mercaptoethanol (βSH), were found to be important features for the proper protein extraction and subsequent analysis. With reference to the transthyretin protein (TTR), the diagnosis of amyloid pathology caused by the same is linked to the recognition of TTR monomer at about 14 kDa after SDS-PAGE and Western-blot electrophoresis. Under normal conditions, the TTR protein circulates in the form of a homotetramer, but with age or due to mutations in the protein sequence, the protein is dissociated in dimers of approximately 20-25 kDa and monomers with molecular weight of about 14 kDa, which, in fact, are then responsible for the formation of fibrils. Figure 3 shows the result of a Western Blot for the TTR protein of samples negative for amyloidosis (control group) extracted with buffers at different pH and with the presence or absence of the reducing agent βSH, representative of 20 extracts.

In the same control group at pH 5.0 and pH 7.2 the Western Blot has identified the presence of TTR protein in tetramer form, dimer form and even in "pathological" form represented by a monomer of about 14 kDa. The monomer was present both in the presence and absence of βSH in the extraction buffer (Figure 3). With strongly basic pH of 8.8 and βSH in the extraction buffer, the Western Blot for the TTR protein shows the pathological monomer. At pH 8.8 without βSH, the Western Blot reveals the presence of tetramer and dimer in the extraction buffer, and the absence of the pathological monomer in the sample negative for amyloid (Figure 3). For k light chains and lambda light chains proteins Western Blot is not changed by the extraction pH and the presence of βSH.

Also the extraction temperature plays an important role in the chemical identification of proteins responsible for the amyloid pathology, because it is able to influence the denaturation of the sample. As for the k and λ light chains, no significant variations were observed about their identification related to different test temperatures. The TTR protein, instead, is more sensitive to temperature, so finding the ideal extraction condition, that does not lead to the formation of the pathological monomer in the negative samples and that highlights the monomer in the positive samples, is critical. The samples negative for amyloid diagnosis, when treated at temperatures of 80°C to 60°C, always showed the presence of the TTR monomer after Western Blot analysis. Treatment of the sample at a temperature of 50°C has allowed, instead, a correct chemical characterization of TTR protein. With this type of procedure the false-pathological monomer does not occur as an artifact in samples negative for amyloidosis but only in samples positive for TTR type amyloidosis (Figure 4). In some cases, protein extracts from FFPE material showed a slight band at about 14 kDa. Analysing the data with the 3D software, the samples negative for TTR type amyloid, however, have not shown the same peak intensity, when compared to the control positive for TTR type amyloid (Figure 4), but rather an intensity that is comparable to the background noise.

### Application of the extraction method to samples stored in RNAlater

8 endomyocardial biopsies preserved in RNAlater of subjects with Immunoelectronmicroscopic characterization for amyloid pathology were selected and treated to be analyzed by SDS-PAGE electrophoresis and Western Blot.

A cold protein extraction was performed without addition of further additives in the buffer. The results obtained from the analysis with Western Blot are superimposable with this immunoelectronmicroscopic characterization, as shown in Table 1.

**Table 1: Comparison of the results of chemical characterization of amyloid protein with two methods**

| **#** | **Time in RNAlater** | **Immunoelectronmicroscopy characterization** | **Western Blot findings** |
|---|---|---|---|
| #1 | <1 year | weak κ and TTR | κ, TTR |
| #2 | 3 years | TTR | κ, λ, TTR |
| #3 | 3 years | λ | κ, λ |
| #4 | 1 year | λ | λ |
| #5 | <1 year | λ | λ |
| #6 | <1 year | κ | κ |
| #7 | 1 year | negative | negative |
| #8 | 2 years | negative | negative |

A representative selection of the chemical characterization findings with SDS-PAGE and Western Blot analysis of endomyocardial biopsies preserved in RNAlater is shown in Figure 5.

### Application of the extraction method to samples stored in OCT snap-freeze

The extraction of samples stored in OCT was performed with the same procedures as for the extraction of samples stored in RNAlater.

### Application of the extraction method to samples stored in FFPE

The samples stored in FFPE, instead, were cold-extracted and subsequently the crude extract was treated at 50°C for a time from two hours to over night.

The FFPE samples extracted at a temperature of 50°C and tested with SDS-PAGE electrophoresis and Western Blot showed results congruent to the diagnosis previously made with immunoelectronmicroscopy (Figure 6), and the presence of false negatives and false positives was not detected.

### Chemical characterization of K light chains and lambda (λ) light chains

Figure 7 shows an example of AL amyloidosis with prevalence of lambda chains. The Western Blot shows a band at about 25 kDa in the tested samples, which corresponds to the molecular weight expected for the lambda type light chains.

Moreover, electrophoresis, as confirmed by Western Blot and immunoelectronmicroscopy, shows negativity for kappa light chains and for TTR protein. In this case, the immunohistochemistry showed positivity for both lambda and kappa light chains, although with a predominance of lambda light chains (Figure 7).

Figure 8 shows instead a case of TTR type amyloidosis. Gel electrophoresis and subsequent Western Blot show a band at about 14 kDa.

Other bands are also visible in the blot at various molecular weights, which are probably due to the formation of TTR aggregates.

The immunoelectronmicroscopy confirms the presence of TTR in the sample under examination.

An examination of the results so obtained and mentioned above shows that the diagnostic method based on electrophoresis and immunoblotting, subject-matter of the invention, fulfills the objects of the invention, having proven reliable and routinely applicable in a clinical analysis laboratory.

In fact, the protein extract from fixed material appears to be comparable in quality to that obtained from the frozen material. This finding is important, considering that the inclusion in paraffin of the sample is a necessary procedure for certain types of investigation and preservation of the sample for further in-depth study. The method can be applied on large scale to all the biopsy samples regardless of their conservation mode.

In addition, unlike the extraction methods known in the literature previously cited, the extraction step of the present method uses a buffer based on the presence of Tris-HCI and SDS in the extraction solution. This makes the sample directly applicable to electrophoresis and subsequent Western Blot, without any further steps for extract purification.

During the study, different conditions were tested for the extraction of proteins, which showed variable characteristics in relation to the buffer pH, the presence of additive agents and the extraction temperatures, in order to identify the ideal extraction protocol. All combinations of these conditions allowed to identify an ideal extraction protocol, which involves the use of a lysis buffer based on Tris-HCI and SDS at pH 8.8, without the addition of reducing agents and with an extraction temperature of 50°C.

The results of SDS-PAGE electrophoresis and immunoblotting for Kappa (κ) and lambda (λ) light chains do not show significant changes about their identification, when subjected to different extraction conditions.

Identification of the amyloidogenic protein that is present in each of the deposits with immunoblotting technique is a simple, fast and accurate analytical approach.

### Mass Spectrometry.

The results obtained by western blot were validated by using the Mass Spectrometry technique, which from a biochemical point of view appears to be the gold standard for the detection and characterization of proteins.

Proteins from 10 patients suffering from amyloidosis were extracted from endomyocardial biopsies, as indicated above.

The total protein extract was run on a one-dimensional electrophoresis with SDS-PAGE, each sample was run double.

The gel was subjected to Western blot analysis as per the above protocol.

The bands relating to the protein of interest were excised from the gel and subjected to mass spectrometry (XEVO G2, QTOF).

### Results:

In all cases the characterization by western blot analysis corresponded to the results obtained by mass spectrometry. Figure 9A shows an example of the Mass Spectrometry experiment that shows the complete matching of the western blot indicated above and the characterization by mass spectrometry.

## Claims

1. A method for identifying amyloidosis, comprising the steps of:
a. contacting a sample of biological material with a lysis buffer having a pH of 8.8, said buffer comprising 65.5 mM Tris-HCL, 10% w/v glycerol, 2.3% w/v SDS, and a 2X protease inhibitor;
b. sonicating the sample of step a. at 4°C for 5 minutes and leaving it to rest for another 5 minutes;
c. repeating step b. at least two further times;
d. centrifuging the sample of step c.;
e. recovering the supernatant resulting from the centrifuging step d., thus allowing to obtain a sample of extracted proteins;
f. separating and analyzing the proteins present in the sample obtained from step e. by means of an electrophoretic technique and a subsequent immunofixation.

2. The method according to claim 1, wherein said biological sample was previously preserved by means of freezing, in an OCT solution, in an RNA stabilizing solution or by means of formalin fixing and paraffin inclusion.

3. The method according to claim 2, wherein said biological sample was previously preserved by means of formalin fixing and paraffin inclusion (FFPE)

4. The method according to anyone of claims 2 or 3., wherein when said biological sample is included in paraffin, step c' of incubating the sample of step c. overnight in a thermostatic bath at 50-52°C is added.

5. The method according to anyone of claims 1 to 4, wherein, before step a. of contacting the sample of biological material with a lysis buffer, the sample is washed in a saline solution to which a protease inhibitor is added.

6. The method according to anyone of claims from 1 to 5, wherein said amyloidosis is cardiac amyloidosis correlated to transthyretin.

7. The method according to anyone of claims from 1 to 6, wherein the electrophoretic technique of said step f. is SDS-PAGE, immunofixation and Western Blot.

8. The method according to anyone of claims 1 to 7, wherein said biological sample is an endomyocardium biopsy, of periumbilical fat.

9. The method according to anyone of claims 1 to 8, wherein the protein typing is carried out by means of the molecular weight analysis and by immunoblot with specific antibodies.

10. A lysis buffer for protein extraction having a pH of 8.8 and comprising 65.5 mM Tris-HCL, 10% w/v glycerol, 2.3% w/v SDS, and a 2X protease inhibitor.

11. A kit comprising at least a container with the lysis buffer, having a pH of 8.8 and comprising 65.5 mM Tris-HCL, 10% w/v glycerol, 2.3% w/v SDS, and a 2X protease inhibitor, and an instructions leaflet.

## Patentansprüche

1. Verfahren zur Identifizierung von Amyloidose, umfassend folgende Schritte:
a. Kontaktieren einer Probe biologischen Materials mit einem Lysepuffer, der einen pH-Wert von 8,8 aufweist, wobei der Puffer 65,5 mM Tris-HCL, 10% w/v Glycerin, 2,3% w/v SDS, und einen 2X-Protease-Inhibitor umfasst;
b. Beschallen der Probe aus Schritt a. bei 4°C für 5 Minuten und weitere 5 Minuten stehen lassen;
c. Wiederholen von Schritt b., mindestens zwei weitere Male;
d. Zentrifugieren der Probe aus Schritt c.;
e. Gewinnen des Überstandes, der sich aus dem Zentrifugationsschritt d. ergibt, wodurch eine Probe extrahierter Proteine erhalten werden kann;
f. Trennen und Analysieren der in der in Schritt e. erhaltenen Probe vorhandenen Proteine mittels einer elektrophoretischen Technik und einer anschließenden Immunofixierung.

2. Verfahren gemäß Anspruch 1, wobei die biologische Probe zuvor durch Einfrieren, in einer OCT-Lösung, in einer RNA-Stabilisierungslösung oder durch Formalinfixierung und Paraffineinschluss konserviert wurde.

3. Verfahren gemäß Anspruch 2, wobei die biologische Probe zuvor mittels Formalinfixierung und Paraffineinschluss (FFPE) konserviert wurde.

4. Verfahren gemäß einem der Ansprüche 2 oder 3, wobei, wenn die biologische Probe in Paraffin eingeschlossen wird, der Schritt c' des Inkubierens der Probe aus Schritt c. über Nacht in einem thermostatischen Bad bei 50-52°C hinzugefügt wird.

5. Verfahren gemäß einem der Ansprüche 1 bis 4, wobei vor Schritt a. des Kontaktierens der Probe biologischen Materials mit einem Lysepuffer die Probe in einer Kochsalzlösung gewaschen wird, der ein Proteaseinhibitor zugesetzt wird.

6. Verfahren gemäß einem der Ansprüche 1 bis 5, wobei die Amyloidose eine mit Transthyretin korrelierte kardiale Amyloidose ist.

7. Verfahren gemäß einem der Ansprüche 1 bis 6, wobei die elektrophoretische Technik des Schrittes f. SDS-PAGE, Immunofixierung und Western Blot ist.

8. Verfahren gemäß einem der Ansprüche 1 bis 7, wobei die biologische Probe eine Endomyokardbiopsie von periumbilikalem Fett ist.

9. Verfahren gemäß einem der Ansprüche 1 bis 8, wobei die Proteintypisierung mittels Molekulargewichtsanalyse und mittels Immunoblot mit spezifischen Antikörpern durchgeführt wird.

10. Lysepuffer für die Proteinextraktion, der einen pH-Wert von 8,8 aufweist, und umfassend 65,5 mM Tris-HCL, 10% w/v Glycerin, 2,3% w/v SDS, und einen 2X-Protease-lnhibitor.

11. Kit, umfassend mindestens einen Behälter mit dem Lysepuffer, der einen pH-Wert von 8,8 aufweist, und umfassend 65,5 mM Tris-HCL, 10% w/v Glycerin, 2,3% w/v SDS, und einen 2X-Protease-lnhibitor, und eine Gebrauchsanweisung.

## Revendications

1. Un procédé pour identifier l'amylose, comprenant les étapes consistant à :
a. mettre en contact un échantillon de matériau biologique avec un tampon de lyse ayant un pH de 8,8, ledit tampon comprenant 65,5 mM de Tris-HCL, 10 % p/v de glycérol, 2,3 % p/v de SDS, et un inhibiteur de protéase 2X ;
b. ultrasoniquer l'échantillon de l'étape a. à 4 °C pendant 5 minutes et le laisser reposer pendant 5 autres minutes ;
c. répéter l'étape b. au moins deux fois supplémentaires ;
d. centrifuger l'échantillon de l'étape c. ;
e. récupérer le surnageant résultant de l'étape d. de centrifugation, ce qui permet ainsi d'obtenir un échantillon de protéines extraites ;
f. séparer et analyser les protéines présentes dans l'échantillon obtenu à partir de l'étape e. au moyen d'une technique électrophorétique et d'une immunofixation ultérieure.

2. Le procédé selon la revendication 1, dans lequel ledit échantillon biologique a été préalablement conservé au moyen d'une congélation, dans une solution OCT, dans une solution de stabilisation de l'ARN ou au moyen d'une fixation au formol et inclusion en paraffine.

3. Le procédé selon la revendication 2, dans lequel ledit échantillon biologique a été préalablement conservé au moyen d'une fixation au formol et inclusion en paraffine (FFPE).

4. Le procédé selon l'une quelconque des revendications 2 ou 3, dans lequel ledit échantillon biologique est inclus en paraffine, l'étape c' consistant à incuber l'échantillon de l'étape c. pendant une nuit dans un bain thermostatique de 50 à 52 °C étant ajoutée.

5. Le procédé selon l'une quelconque des revendications 1 à 4, dans lequel, avant l'étape a. consistant à mettre en contact l'échantillon de matériau biologique avec un tampon de lyse, l'échantillon est lavé dans une solution saline à laquelle un inhibiteur de protéase est ajouté.

6. Le procédé selon l'une quelconque des revendications de 1 à 5, dans lequel ladite amylose est une amylose cardiaque corrélée à la transthyrétine.

7. Le procédé selon l'une quelconque des revendications de 1 à 6, dans lequel la technique électrophorétique de ladite étape f. est la SDS-PAGE, l'immunofixation et le Western Blot.

8. Le procédé selon l'une quelconque des revendications 1 à 7, dans lequel ledit échantillon biologique est une biopsie endomyocardique, de graisse périombilicale.

9. Le procédé selon l'une quelconque des revendications 1 à 8, dans lequel le typage de protéines est réalisé au moyen de l'analyse de poids moléculaire et par immunobuvardage avec des anticorps spécifiques.

10. Un tampon de lyse pour l'extraction de protéines ayant un pH de 8,8 et comprenant 65,5 mM de Tris-HCL, 10 % p/v de glycérol, 2,3 % p/v de SDS, et un inhibiteur de protéase 2X.

11. Un kit comprenant au moins un contenant avec le tampon de lyse, ayant un pH de 8,8 et comprenant 65,5 mM de Tris-HCL, 10 % p/v de glycérol, 2,3 % p/v de SDS, et un inhibiteur de protéase 2X, et un mode d'emploi.
